# EUROPEAN PATENT APPLICATION

(11) **EP 1 521 200 A2**
(43) Date of publication of application: **06.04.2005**
(21) Application number: 04010227.9
(22) Date of filing: 29.04.2004
(51) Int. Cl.: G06F 19/00

(54) **Medical data providing system and medical data providing method**

(30) Priority: 02.10.2003 KR 2003068612
(71) Applicant: Samsung Electronics Co., Ltd., Suwon-si, Gyeonggi-do (KR)
(72) Inventor: Kwon, Tae-joon, Bundang-gu Seongnam-si Gyeonggi-do (KR)
(74) Representative: Grünecker, Kinkeldey, Stockmair & Schwanhäusser Anwaltssozietät

(57) **Abstract**

There are provided medical data providing system and method which separate ID (identification) data and non-ID data of a patient to provide only medical data of the patient. The system is comprised an ID information storage unit, an non-ID information storage unit, a medical care code generating unit, a care database and medical care code list database. To epidemiological research, the system is connected to the hospital or research center. When the medical data of a patient are intended to use for medical researches, only the non-ID data are provided to the hospital side in a state where the ID data of the patient exist only in the patient terminal physically separated from a computer system of a hospital, so that it is possible to prevent the personal information of the patient from being leaked.

## Description

### BACKGROUND OF THE INVENTION

### 1. Field of the Invention

The present invention relates to medical data management system, and more specifically, to a medical data providing system and a medical data providing method for protecting personal information and supporting medical researches.

### 2. Description of the Related Art

Patient data such as care data, prescription data, medication data, etc. are frequently exchanged between medical personnel, medical institutions, pharmacies, and the like by using paper records or via the Internet. In the latter case, the patient data are organized into databases, thereby allowing interested parties to easily access and retrieve required medical information.

However, although the patient data can be efficiently handled via the Internet, patient personal information can be drained out and various preservation problems such as privacy invasion, medical data outflow, etc., can appear.

In general, medical data of a patient include personal information data such as the name, sex, address, phone number, resident registration number (social security number in the U.S.), insurance number, birth date, etc., and specific medical data such as care date, care institution, care item, case history, care data, image data, measurement data, prescription data, etc. When the medical data is organized into databases and exchanged through a communication network, a medical data providing method and a medical data sharing system for efficiently protecting data transfer is required. In this regard, U.S. Patent Application Publication No. 2002/0035485 discloses a medical data providing method comprising the steps of: registering data on a peculiar physical feature read from a patient, such as a fingerprint, voice, eye iris or eye fundus image, etc., together with medical data of the patient in a database connected to a server as a medical data source; sending a request for the medical data of the patient to the server as the medical data source through a communication network from a terminal requiring the medical data; transmitting the data on the peculiar physical feature read from the patient; collating the peculiar physical feature data transmitted from the terminal with the physical feature data stored in the database, and transmitting the medical data of the patient stored in the database to the terminal through the communication network from the sever when a collation result indicates a match. The medical data providing method further comprises a step of authenticating whether a requester demanding the medical data is a person previously authorized to request the medical data by using the data on the peculiar physical feature read from the requester.

As a result, the above method allows medical data of patients to be read in the presence of the patients or other authorized persons, so that the medical data can be shared while protecting the personal information of the patients.

On the other hand, the medical data may be used for researches for improving medical technologies. Accordingly, institutions such as hospitals, universities, cancer centers, professional medical research centers, medical societies, etc., are more interested in the specific medical data than in the personal information of the patients to develop medical statistics and prevention, diagnosis, and care technologies and methods on the basis.

However, when the medical data are acquired using the method disclosed in U.S. Patent Application Publication No. 2002/0035485, the specific medical data of the patients are provided together with the personal information data which may lead to personal information drains.

Therefore, there is a need for a medical data providing system and method, which allow medical data to be safely exchanged for medical researches with not disclosing and efficiently protecting the personal information of patients.

### SUMMARY OF THE INVENTION

Accordingly, the present invention provides a medical data providing system which separates personal information and medical care data to provide the medical data.

The present invention also provides a medical data providing method for the medical data providing system.

According to an aspect of the present invention, there is provided a medical data providing system which separates ID (identification) data and non-ID data of a patient to provide only medical data of the patient, the system comprising: an ID storage unit provided in a patient terminal of the patient, the ID storage unit storing the ID data of the patient; a non-ID storage unit provided in the patient terminal, the non-ID storage unit storing the non-ID data of the patient; a medical care code generating unit provided in a hospital server in order to generate medical care codes of the patient; a care database provided in the hospital server, the care database storing care data of the patient; a research database provided in the hospital server, the research database storing the non-ID data of the patient and research data using the medical care codes and the care data of the patient; and a medical care code list database provided in the patient terminal and the hospital server, the care cods list database storing the medical care codes.

The medical data providing system may further comprise a personal information management server which includes the ID storage unit, the medical care code list database, and a medical care code-ID relation database that links the medical care codes with the ID data. The patient terminal may further include an insurance database storing an insurance institution name, an insurance number and an insurance condition of a medical insurance of the patient. The ID storage unit may store personal information including a name, a birth date, an address, a resident registration number, and an insurance number of the patient. The non-ID storage unit may keep non-ID data including a sex, an age, an occupation, etc. of the patient. The research database may be used to epidemiological research and store statistical data such as disease data for sexes, disease data for ages and disease data for occupations, etc.

According to another aspect of the present invention, there is provided a medical data providing method using the medical data providing system according to an aspect of the present invention, the method comprising the steps of: (a) connecting the patient terminal to the hospital server; (b) confirming the ID data and the insurance condition of the patient; (c) storing the medical care codes generated from the hospital server in the patient terminal; (d) giving medical care to the patient in the hospital; and (e) storing the medical care codes and the care data in the medical care code list database and the care database of the hospital server.

According to still another aspect of the present invention, there is provided a medical data providing method using the medical data providing system according to an aspect of the present invention, the method comprising the steps of: (a) requesting the medical data of the patient for the purpose of a medical research; (b) making up a written consent that the patient allows his medical data to be used for the medical research; (c) confirming the non-ID data of the patient, and giving the medical care codes to care samples obtained in the course of care of the patient; and (d) storing the care data of the patient together with the medical care codes in the medical care code list database and the care database of the hospital server.

According to still another aspect of the present invention, there is provided a medical data providing method using the medical data providing system according to an aspect of the present invention, the method comprising the steps of: (a) the patient visiting an emergency room without his patient terminal; (b) acquiring the personal information from the patient, generating the medical care codes, and then giving medical care to the patient in the hospital; (c) storing the medical care codes and the care data of the patient in the medical care code list database and the care database of the hospital server; (d) connecting the patient terminal to the hospital server; and (e) confirming the ID data of the patient, storing the medical care codes of the patient in the patient terminal, and then disconnecting the patient terminal from the hospital server.

According to still another aspect of the present invention, there is provided a medical data providing method using the medical data providing system according to an aspect of the present invention, the method comprising the steps of: (a) notifying an insurance institution of the medical care codes and care details required for demanding a medical insurance money; (b) determining the insurance money in the insurance institution, and providing the insurance money to the hospital to pay off an medical expense; (c) notifying the personal information management server of the medical care codes and the insurance money details; and (d) notifying the patient terminal of the medical care codes and the insurance money details, and recording details associated with insurance in the insurance database of the patient terminal.

As a result, according to the present invention, when the medical data of a patient are intended to use for medical researches, only the non-ID data are provided to the hospital side in a state where the ID data of the patient exist only in the patient terminal physically separated from a computer system of a hospital, so that it is possible to prevent the personal information of the patient from being leaked.

### BRIEF DESCRIPTION OF THE DRAWINGS

The above and other features and advantages of the present invention will become more apparent from detailed description of exemplary embodiments thereof with reference to the attached drawings in which:
FIG. 1 is a block diagram illustrating a medical data providing system according to an embodiment of the present invention;
FIG. 2 is a flowchart illustrating medical care progress of a patient by using the medical data providing system of the present invention;
FIG. 3 is a flowchart illustrating a method of sharing medical data of a patient by using the medical data providing system of the present invention;
FIG. 4 is a flowchart illustrating a procedure corresponding to an emergency where a patient visits an emergency room without his terminal in the medical data providing system of the present invention; and
FIG. 5 is a flowchart illustrating a procedure of demanding a medical insurance money in the medical data providing system of the present invention.

### DETAILED DESCRIPTION OF THE INVENTION

The present invention and operational advantages thereof can be fully understood by referring to the attached drawings and explanations thereof.

Now, exemplary embodiments of the present invention will be described in detail with reference to the attached drawings. In the drawings, the same reference numerals indicate the same elements.

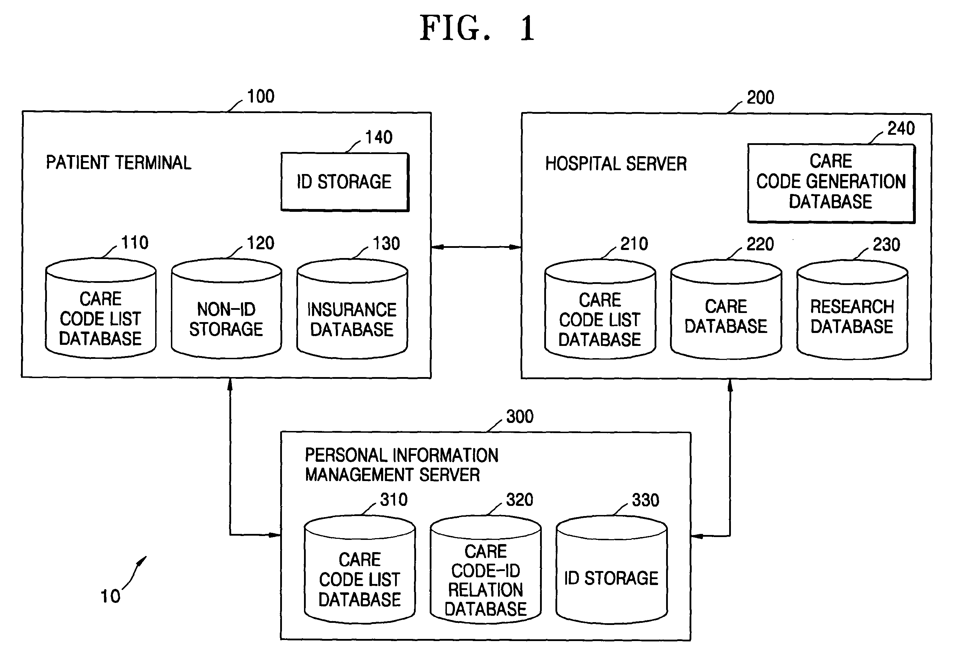

FIG. 1 is a block diagram illustrating a medical data providing system according to an embodiment of the present invention.

In FIG. 1, the medical data providing system 10 comprises a patient terminal 100, a hospital server 200 and a personal information management server 300. The patient terminal 100 includes a medical care code list database 110, a non-identification (ID) database 120, an insurance database 130, and an ID storage unit 140. The hospital server 200 includes a medical care code list database 210, a care database 220, a research database 230, and a medical care code generating unit 240. The personal information management server 300 includes a medical care code list database 310, a medical care code-ID relation database 320, and an ID storage unit 330.

Peculiar personal information such as a name, an address, a phone number, a resident registration number (social security number in U.S.), an insurance number, a birth date, etc. of a patient is stored in the ID storage unit 140. Data in the personal information such as sex, age, occupation, etc. of the patient, which are available for researches but cannot identify the patient, are stored in the non-ID storage unit 120. Medical insurance conditions such as insurance institution, insurance policy number, etc., which the patient joins in, are stored in the insurance database 130.

The medical care code generating unit 240 codes care details given to the patient in the hospital, that is, hospital name, care date, care item, doctor name, inspection date, inspection name, inspection result, etc., and generates medical care codes. It is preferable that the medical care codes are provided as standardized codes, which can be shared through communication networks by another hospital or research institutions. The medical care codes generated from the medical care code generating unit 240 are stored in the respective medical care code list databases 110, 210, 310 of, the patient terminal 100, the hospital server 200, and the personal information management server 300.

Care records given to the patient in the hospital, that is, care history data such as diagnosis, judgment, prescription, etc. and emergency data such as blood group, histocompatibility type, allowed drugs, contraindicated drugs, functional details of the internal organs, etc. are stored in the care database 220 in conjunction with the medical care codes. Medical data for developing disease prevention programs, disease diagnosis programs and disease remedy programs on the basis of the medical care codes stored in the medical care code list database 210 are stored in the research database 230. In addition, statistical data of disease data for sexes, disease data for ages and disease data for occupations prepared by using the medical care codes stored in the medical care code list database 210 are stored in the research database 230.

The same data as stored in the ID storage unit 140 of the patient terminal 100 are stored in the ID storage unit 330 of the personal information management server 300. The medical care code-ID relation database 320 links the personal information stored in the ID storage unit 330 with the medical care codes stored in the medical care code list data base 310 to provide the medical data to another hospital requiring the care data of the patient. The medical care code-ID relation database 320 needs a preservation system to prevent the personal information from draining out.

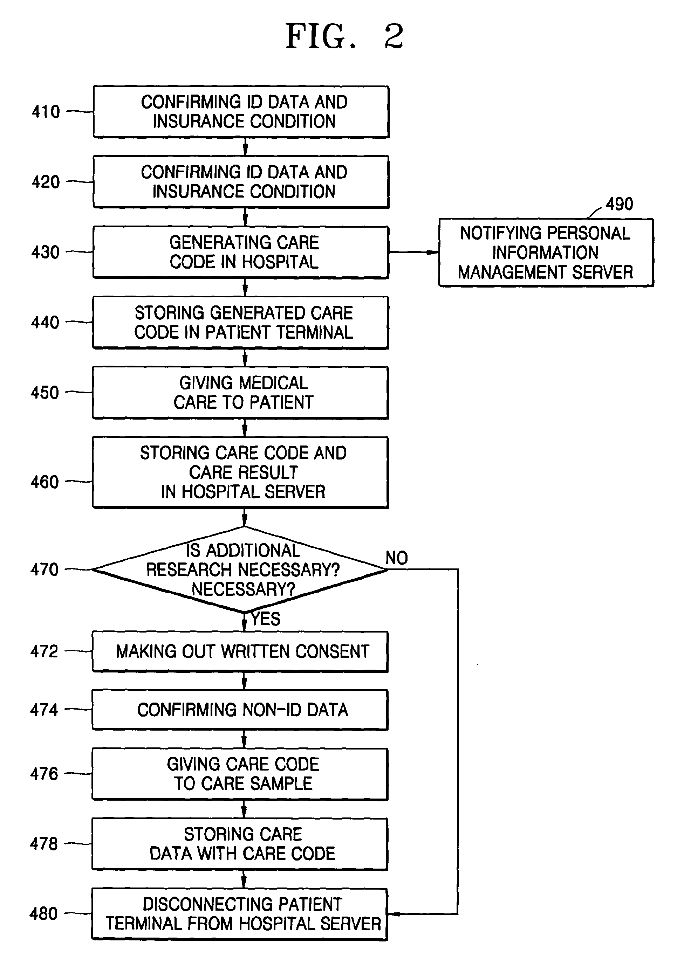

FIG. 2 is a flowchart illustrating medical care progress of a patient by using the medical data providing system 10 of the present invention. Referring to FIG. 2, when a patient connects to a hospital with his patient terminal 100 (410), the ID data and the insurance conditions of the patient are confirmed (420). When the medical care codes 430 are generated in the hospital (430), the generated medical care codes are notified to the personal information management server 300 (490), and stored in the patient terminal 100 (440). Then, the patient is subjected to the medical care in the hospital (450), and the medical care codes and a care result are stored in the databases 210, 220 (FIG. 1) of the hospital server 200 (460). When the care data of the patient are requested for the medical research (470), the patient makes up a written consent representing that his medical data may be used for the medical research (472). Then, the non-ID data of the patient is confirmed (474), medical care codes are given to care samples obtained in the course of the medical care (476), and then the care data are stored together with the medical care codes (478). Finally, the patient disconnects his patient terminal 100 (480).

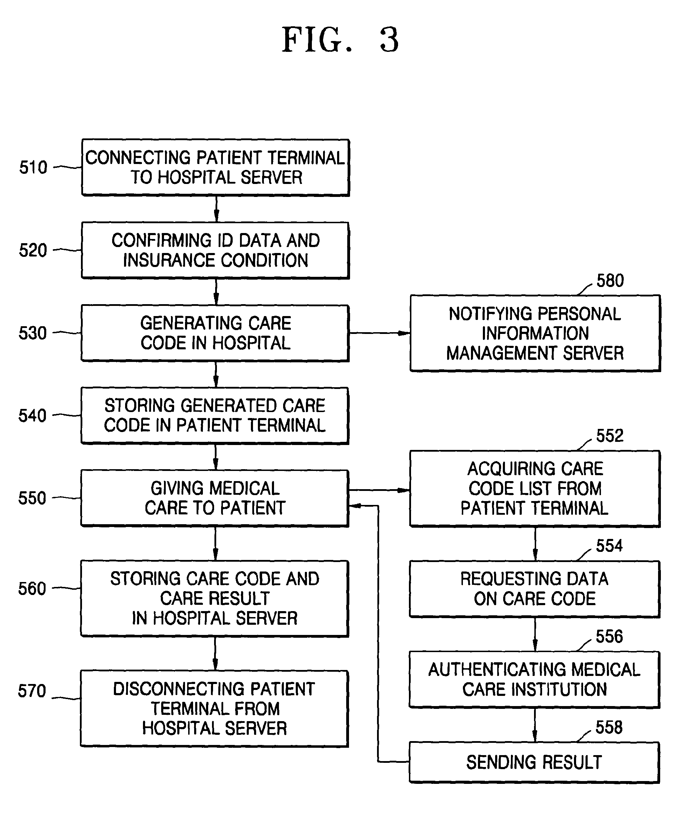

FIG. 3 is a flowchart illustrating a method of sharing medical data of a patient by using the medical data providing system 10 of the present invention. Referring to FIG. 3, a patient connects to the hospital server 200 with his patient terminal 100 (510), and the ID data and an insurance condition of the patient are confirmed (520). When a medical care code is generated in the hospital (530), the generated medical care code is notified to the personal information management server 300 (580), and stored in the patient terminal 100 (540). Then, a medical care is given to the patient in the hospital (450). At this time, when a list of the generated medical care codes is acquired (552) and data on the medical care code is requested (554), care result data is notified to an institution having requested the data on the medical care codes (558), via an authentication procedure in the hospital server 200 receiving the request of data on the medical care codes (556). The care data of the patient are stored together with the medical care codes in the databases 210, 220 (FIG. 1) of the hospital server 200 (560). Then, the patient disconnects the patient terminal 100 from the hospital server 200 (570).

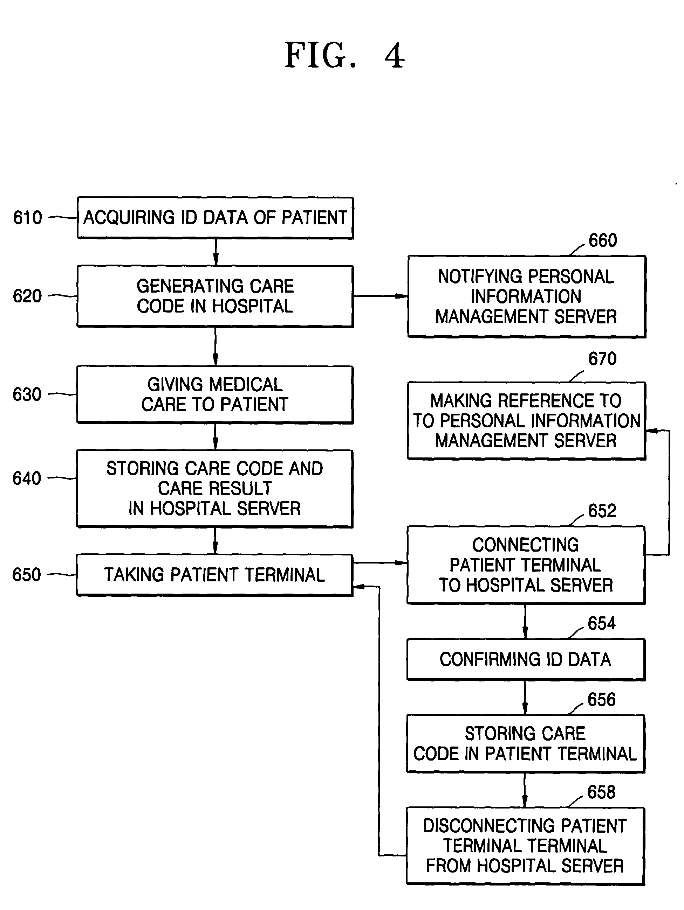

FIG. 4 is a flowchart illustrating a procedure corresponding to an emergency where a patient visits an emergency room without his terminal 100 in the medical data providing system 10 of the present invention. Referring to FIG. 4, the personal information is acquired from the patient (610), the medical care codes are generated in the hospital (620), and then a medical care is given to the patient (630). The medical care codes and the care data of the patient are stored in the databases 210, 220 (FIG. 1) of the hospital server 200 (640). When the patient carrying the patient terminal 100 (650) connects to the hospital server 200 with the patient terminal 100 (652), the ID data of the patient is confirmed (654), the medical care codes of the patient are stored in the patient terminal 100 (656), and then the patient terminal 100 is disconnected from the hospital server 200 (658).

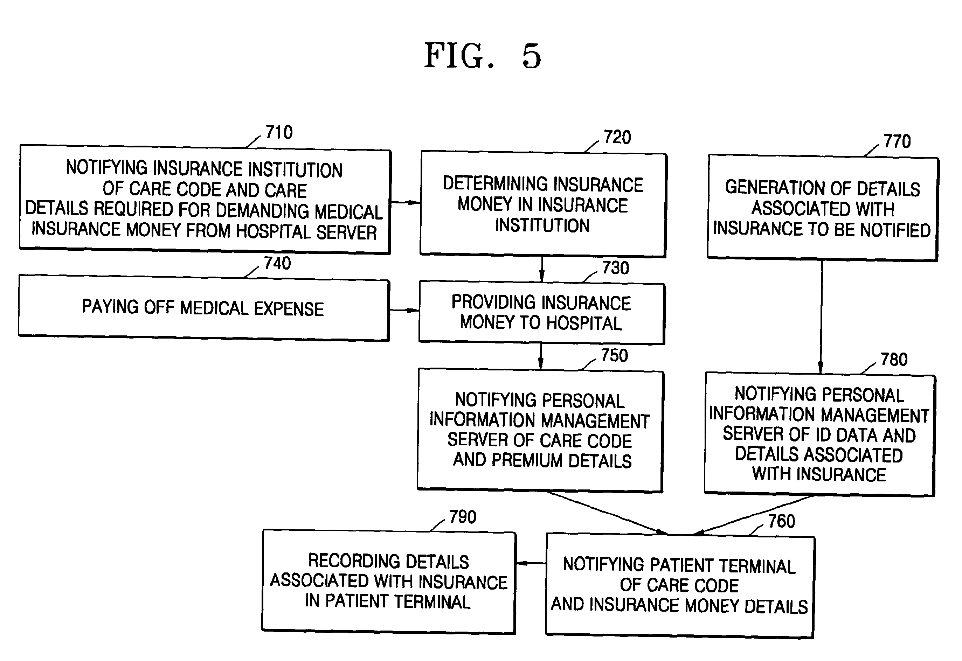

FIG. 5 is a flowchart illustrating a procedure of demanding a medical insurance money in the medical data providing system 10 of the present invention. Referring to FIG. 5, when the medical care codes and care details required for demanding a medical insurance money are notified to an insurance institution from the hospital server 200 (710), the insurance money is determined in the insurance institution (720), and the insurance money is provided to the hospital (730) to pay off an medical expense (740). Then, the medical care codes and the insurance money details are notified to the personal information management server 300 (FIG. 1) (750). When details associated with insurance to be notified are generated in an institution other than the hospital (770), the ID data and the details associated with insurance are notified to the personal information management server 300 (FIG. 1) (780). Then, the medical care codes, the insurance money details and the details associated with insurance are notified to the patient terminal 100 (760), and the details associated with insurance are recorded in the insurance database 130 (FIG. 1 ) (790).

As a result, according to the medical data providing system of the present invention, when the medical data of a patient are demanded to use for medical researches, the non-ID data separated from the ID data of the patient are provided together with the medical care codes. Therefore, it is possible to prevent the personal information of the patient from being leaked.

While the present invention has been particularly shown and described with reference to exemplary embodiments thereof, it will be understood by those skilled in the art that various changes in form and details may be made therein without departing from the spirit and scope of the present invention as defined by the following claims.

## Claims

1. A medical data providing system which separates ID (identification) data and non-ID data of a patient to provide only medical data of the patient, the system comprising:
an ID storage unit provided in a patient terminal of the patient, the ID storage unit storing the ID data of the patient;
a non-ID storage unit provided in the patient terminal, the non-ID storage unit storing the non-ID data of the patient;
a medical care code generating unit provided in a hospital server in order to generate medical care codes of the patient;
a care database provided in the hospital server, the care database storing care data of the patient; and
a medical care code list database provided in the patient terminal and the hospital server, the medical care code list database storing the medical care codes.

2. The medical data providing system according to claim 1, further comprising a research database provided in the hospital server, the research database storing the non-ID data of the patient and research data using the medical care codes and the care data of the patient; and

3. The medical data providing system according to claim 1, further comprising a personal information management server which includes the ID storage unit, the medical care code list database, and a medical care code-ID relation database that links the medical care codes with the ID data.

4. The medical data providing system according to claim 1, wherein the patient terminal further includes an insurance database storing an insurance institution name, an insurance number and an insurance condition of a medical insurance of the patient.

5. The medical data providing system according to claim 1, wherein the ID storage unit stores personal information including a name, a birth date, an address, a resident registration number, and an insurance number of the patient.

6. The medical data providing system according to claim 1, wherein the non-ID storage unit stores non-ID data including a sex, an age and an occupation of the patient.

7. The medical data providing system according to claim 2, wherein the research database stores statistical data such as disease data for sexes, disease data for ages and disease data for occupations, etc. prepared by using the medical care codes stored in the medical care code list database.

8. A medical data sharing method using the medical data providing system according to any one of claims 1 through 5, the method comprising the steps of:
(a) connecting the patient terminal to the hospital server;
(b) confirming the ID data of the patient;
(c) storing the medical care codes generated from the hospital server in the patient terminal;
(d) giving medical care to the patient in the hospital; and
(e) storing the medical care codes and the care data in the medical care code list database and the care database of the hospital server.

9. The medical data sharing method according to claim 8, further comprising steps of
(f) acquiring a list of the generated medical care codes from the patient terminal;
(g) requesting data on the medical care codes from the hospital having the generated medical care codes;
(h) notifying care result data to an institution having requested the data on the medical care codes via an authentication procedure in the hospital server; and
(i) receiving the request of data on the medical care codes to the hospital requesting the medical care codes.

10. A medical data providing method using the medical data providing system according to any one of claims 1 through 5, the method comprising the steps of:
(a) requesting the medical data of the patient for the purpose of a medical research;
(b) making up a written consent that the patient allows his medical data to be used for the medical research;
(c) confirming the non-ID data of the patient, and giving the medical care codes to care samples obtained in the course of care of the patient; and
(d) storing the care data of the patient together with the medical care codes in the medical care code list database and the care database of the hospital server.

11. The medical data providing method according to claim 10, wherein the written consent is made up to represent that the medical data of the patient are used for the research for a predetermined time.

12. The medical data providing method according to claim 10, further comprising a step of (e) notifying the personal information management server of the medical care codes.

13. A medical data providing method using the medical data providing system according to any one of claims 1 through 5, the method comprising the steps of:
(a) the patient visiting an emergency room without his patient terminal;
(b) acquiring the personal information from the patient, generating the medical care codes, and then giving medical care to the patient in the hospital;
(c) storing the medical care codes and the care data of the patient in the medical care code list database and the care database of the hospital server;
(d) connecting the patient terminal to the hospital server; and
(e) confirming the ID data of the patient, storing the medical care codes of the patient in the patient terminal, and then disconnecting the patient terminal from the hospital server.

14. A medical data providing method using the medical data providing system according to any one of claims 1 through 5, the method comprising the steps of:
(a) notifying an insurance institution of the medical care codes and care details required for demanding a medical insurance money;
(b) determining the insurance money in the insurance institution, and providing the premium to the hospital to pay off a medical expense;
(c) notifying the personal information management server of the medical care codes and the insurance money details; and
(d) notifying the patient terminal of the medical care codes and the insurance money details, and recording details associated with insurance in the insurance database of the patient terminal.

15. The medical data providing method according to claim 14, further comprising a step of (e) when details associated with insurance to be notified are generated in an institution other than the hospital, notifying the personal information management server of the ID data and the details associated with insurance.
